# EUROPÄISCHE PATENTSCHRIFT

(11) **EP 0 848 066 B1**
(45) Veröffentlichungstag und Bekanntmachung des Hinweises auf die Patenterteilung: **12.11.2003**
(21) Anmeldenummer: 97121272.5
(22) Anmeldetag: 04.12.1997
(51) Int. Cl.: C12Q 1/42, C12Q 1/44

(54) **Mittel und Verfahren zur Bestimmung von hydrolytischen Enzymen**
Means and method for determining hydrolytic enzymes
Moyen et procédé pour la détection des enzymes hydrolytiques

(30) Priorität: 13.12.1996 DE 19651886
(43) Veröffentlichungstag der Anmeldung: 17.06.1998
(73) Patentinhaber: MERCK PATENT GmbH, 64271 Darmstadt (DE)
(72) Erfinder: Linxweiler, Winfried Dr., 64823 Gross-Umstadt (DE)

(56) Entgegenhaltungen:
- EP-A- 0 237 394
- EP-A- 0 290 217
- EP-A- 0 381 173
- DE-A- 3 000 292
- GB-A- 1 128 371
- US-A- 3 699 005
- US-A- 4 278 763
- US-A- 4 892 817

## Beschreibung

Die Erfindung betrifft Mittel und Verfahren zur Bestimmung von hydrolytischen Enzymen in flüssigen Proben, z.B. in Lebensmitteln und biologischen Lösungen mit Hilfe von festen Reagenzienträgern und quantitativer Bestimmung mittels Reflektometrie.

Hydrolytische Enzyme in Lebensmitteln stammen entweder aus dem Lebensmittel selbst oder von Mikroorganismen, welche die Lebensmittel kontaminieren. Diese Enzyme sind oft die Ursache für den Lebensmittelverderb, besonders wenn die Enzyme durch die Methoden der Entkeimung der Lebensmittel nicht vollständig inaktiviert werden. Solche Hydrolasen sind z.B. Lipasen, Ali- und Arylesterasen, Phosphatasen, Sulfatasen, Glucuronidasen, Glucosidasen, Amylasen und Proteinasen. Hydrolytische Enzyme in biologischen Lösungen wie Blut, Plasma, Serum, Urin usw. sind z.B. Lipase, Amylase, Proteinasen, Cholinesterasen und Phosphatasen. Eine Änderung ihrer Konzentration kann Krankheitszustände oder bestimmte physiologische Zustände anzeigen. Die schnelle und empfindliche Bestimmung der Konzentration ist für die Diagnose dieser Zustände wichtig.

Die Bestimmung hydrolytischer Enzyme mit Hilfe von saugfähigen Trägern, die sowohl einen Indoxylester als auch ein Oxidationsmittel zusammen auf einem Träger enthalten, sind aus GB 1128371 und EP 12957 bekannt. Aus Nahrung 9, 445 (1965) ist ein Testpapier zum Nachweis von Esterasen in tierischen und pflanzlichen Geweben und Mikroorganismen bekannt, das Indoxylacetat enthält. Als Oxidationsmittel dient Luftsauerstoff. Die Zeitspanne bis zum Auftreten einer Farbreaktion läßt eine Abschätzung der Esterasekonzentration zu.

US 4,278,763 offenbart diagnostische Reagenzzusammenstellungen für die Proteasebestimmung, wobei Indoxylaminosäureester oder -peptidester als Substrate verwendet werden. GB 1 128 371 offenbart Verfahren und Teststreifen zur Messung der Aktivität hydrolytischer Enzyme, wobei Indoxylderivate als Substrate in Gegenwart eines Oxidationsmittels verwendet werden. DE 30 00 292 A1 offenbart Indoxylmaltodextrine und deren Verwendung als Substrat für die Amylasebestimmung.

Der Nachteil der bekannten Verfahren liegt in der begrenzten Empfindlichkeit, insbesondere bei Proben mit hohem Fett-, Eiweiß-, Salz- und Fremdstoffgehalt. Außerdem kann bei den visuell auszuwertenden Verfahren die Enzymaktivität nur abgeschätzt werden.

Der Erfindung liegt die Aufgabe zugrunde, ein Mittel und Verfahren zur Bestimmung von hydrolytischen Enzymen zur Verfügung zu stellen, mit dem wesentlich höhere Empfindlichkeiten im Vergleich zum Stand der Technik erreicht werden und das quantitative Bestimmungen ermöglicht.

Überraschenderweise wurde gefunden, daß durch das Vorliegen des Oxidationsmittels in einer gesonderten Lösung und durch das Eintauchen des die Indoxylverbindung enthaltenden saugfähigen Trägers in diese Lösung eine um den Faktor 3-12 höhere Empfindlichkeit erreicht wird, als das bisher möglich war. Ein weiterer Vorteil ist die größere Haltbarkeit, die mit der Trennung der Reaktionspartner verbunden ist, sowie die geringe Menge Substrat, die benötigt wird.

Gegenstand der Erfindung ist ein Verfahren zur Bestimmung von hydrolytischen Enzymen in flüssigen Proben mit folgenden Verfahrensschritten:
a) Benetzen eines mit einer Indoxylverbindung imprägnierten saugfähigen Trägers mit der Probelösung;
b) Eintauchen des Trägers aus Schritt a) in eine ein Oxidationsmittel enthaltende Lösung;
c) Inkubation des Trägers;
d) reflektometrische Auswertung des Trägers;

Gegenstand der Erfindung ist weiterhin ein Mittel zur Bestimmung von hydrolytischen Enzymen in flüssigen Proben nach dem obigen Verfahren bestehend aus
- einem saugfähigen Träger, der mit einer Indoxylverbindung imprägniert ist, sowie
- einer Lösung, die ein Tetrazoliumsalz als Oxidationsmittel enthält.

Die auf dem saugfähigen Träger vorliegenden Indoxylverbindungen sind spezifische Enzymsubstrate für die hydrolytischen Enzyme. Geeignete Indoxylverbindungen sind z.B. Indoxylester mit 2-20 C-Atomen im Esterteil wie Indoxylacetat, -butyrat, -caprylat, -palmitat, Indoxylglucuronid, -galactosid, -maltotriose, -phosphat, -sulfat, vorzugsweise mit Halogen substituierte Indoxylester wie 5-Bromindoxyl-, 6-Chlorindoxyl-, 5-Brom-6-chlorindoxyl-, 5-Brom-4-chlorindoxylester, vorzugsweise 5-Brom-4-chlor-3-indoxylcaprylat, 5-Brom-4-chlor-indoxylphosphat und Indoxylacetat.

Als saugfähige Träger können alle verwendet werden, die üblicherweise für solche Tests im Gebrauch sind. Am weitesten verbreitet ist die Verwendung von Filterpapier, jedoch können auch andere saugfähige Cellulose- oder Kunststoffprodukte eingesetzt werden. Die saugfähigen Träger, vorzugsweise Filterpapier, werden in an sich bekannter Weise mit Tränklösungen imprägniert, die die Indoxylverbindungen enthalten. Die getränkten und getrockneten Papiere können zu quadratischen bzw. rechteckigen Zonen verarbeitet werden, die ihrerseits in bekannter Weise auf Kunststoffolien, Papier- oder Metallstreifen aufgeklebt bzw. aufgesiegelt werden können.

Die saugfähigen Träger können auch vor dem Imprägnieren in Streifenform auf ein Kunststoffband aufgebracht und nach dem Imprägnieren senkrecht zur Streifenrichtung in handliche Stäbchen geschnitten werden.

Die Tränklösung enthält etwa 0,005-2 g, vorzugsweise 0,05 g der entsprechenden Indoxylverbindung in 100 ml eines organischen Lösungsmittel, z.B. in Ethanol, Methanol, Aceton. Gegebenenfalls kann die Tränklösung außerdem noch Wasser, Puffersubstanzen, Komplexbildner und Katalysatoren enthalten. Das Testpapier wird vorteilhafterweise lichtund feuchtigkeitsgeschützt bei 4-8 °C gelagert.

Bei den in Lösung vorliegenden Oxidationsmitteln handelt es sich um Tetrazoliumsalze wie Nitroblautetrazoliumchlorid, Tetranitroblautetrazoliumchlorid (TNBT), Dimethylthiazolyldiphenyltetrazoliumbromid (MTT), Tetrazoliumblauchlorid, Triphenyltetrazoliumchlorid, Jodphenylnitrophenylphenyltetrazoliumchlorid (INT), Neotetrazoliumchlorid, Phenylaminocarbonyltetrazolium-bismethoxynitrobenzolsulfonsäure-Natriumsalz (XTT), vorzugsweise Nitroblautetrazoliumchlorid (NBT). Die Tetrazoliumsalze werden in einem geeigneten Lösungsmittel, vorzugsweise Ethanol, gelöst und mit Wasser auf eine Konzentration von 0,02-10 mg/ml, vorzugsweise 1 mg/ml, eingestellt. Auch diese Lösung kann noch stabilisierende Zusätze enthalten, z.B. Detergenzien. Das Verhältnis von Indoxylverbindung zu Oxidationsmittel ist nicht kritisch; es sollte im Bereich von 5 : 1 bis 1 : 20, vorzugsweise bei etwa 1 : 2 liegen.

Zur Durchführung der Enzymbestimmung wird die Probe entweder direkt eingesetzt, mit einem Puffer verdünnt oder extrahiert. Die Puffersubstanzen sollen einen pH-Bereich von 6-11, vorzugsweise einen pH-Wert von etwa 8 aufrechterhalten können. Die einzusetzende Pufferkonzentration richtet sich nach dem pH-Wert der Probelösung. Als Puffer eignen sich z.B. Tris/HCL-Puffer, HEPES-Puffer, MOPS-Puffer, Natronlauge/Tartrat-Puffer, Natronlauge/Borat-Puffer, Natriumcarbonat/-Natriumhydrogencarbonat-Puffer, vorzugsweise Tris/HCL-Puffer.

Der saugfähige Träger wird etwa 2 Sekunden in die Probelösung eingetaucht, kräftig abgeschüttelt oder abgestreift und so in die Tetrazoliumsalzlösung getaucht, daß die Reaktionszone vollständig benetzt ist. Die Tauchzeit liegt zwischen 1 und 60 Minuten, vorzugsweise bei etwa 15 Minuten. Der Träger wird dann aus der Probelösung entfernt, kräftig abgeschüttelt und die entstandene Färbung wird mit einem Reflektometer (340-700 nm) oder anhand einer Farbvergleichsskala ausgewertet. Mit dem erfindungsgemäßen Verfahren ist eine schnelle, genaue und hochempfindliche Bestimmung hydrolytischer Enzyme, insbesondere von Lipasen, Esterasen, Phosphatasen und Sulfatasen möglich.

### Beispiel 1

### Bestimmung von Lipase

### a) Herstellung des Testpapiers

Ein Filterpaier (Schöller & Hösch 300A) wird mit einer Lösung von 56 mg 5-Brom-4-chlor-3-indoxylcaprylat in 112 ml Ethanol (96 %) getränkt und nach der Tränkung getrocknet. Das erhaltene Papier wird auf ein Trägermaterial, z.B. Polyesterfolie, in bekannter Weise aufgebracht.

### b) Herstellung der Oxidationsmittellösung

Die Oxidationsmittellösung enthält 60 mg Nitroblautetrazoliumchlorid, das in 12,5 ml Ethanol (96 %) gelöst wird. Diese Lösung wird zu 49,3 ml Wasser gegeben, das 1,5 g Detergens (Tween 20) enthält.

### c) Herstellung der Lipasestandardlösungen

Mit Lipoproteinlipase aus Pseudomonas sp. werden durch Verdünnen mit einer Pufferlösung enthaltend

| | |
|---|---|
| 500 mmol/l | Tris/HCl-Puffer, pH 8,0 |
| 4 mmol/l | Magnesiumchlorid |
| 2 mmol/l | EDTA |
| 0,1 mg/ml | Rinderserumalbumin und |
| 2 % | Tween 20 |

Lipasestandardlösungen in den Konzentrationen 10, 20, 60, 120, 200, 300, 450 und 600 µg/l hergestellt.

### d) Bestimmung der Lipase

Das Teststäbchen wird für etwa 2 Sekunden in die Enzymlösung eingetaucht, abgeschüttelt und 15 Minuten in etwa 1 ml der Oxidationsmittellösung eingetacht. Nach dieser Zeit hat sich eine blaue Farbe entwickelt, die in einem Reflektometer (576 nm) gemessen wird:

| | | | | | | | | |
|---|---|---|---|---|---|---|---|---|
| Konz. (µg/l) | 10 | 20 | 60 | 120 | 200 | 300 | 450 | 600 |
| relative Rem. (%) | 71,4 | 68,4 | 60,8 | 53,9 | 48,7 | 43,3 | 38 | 32,2 |

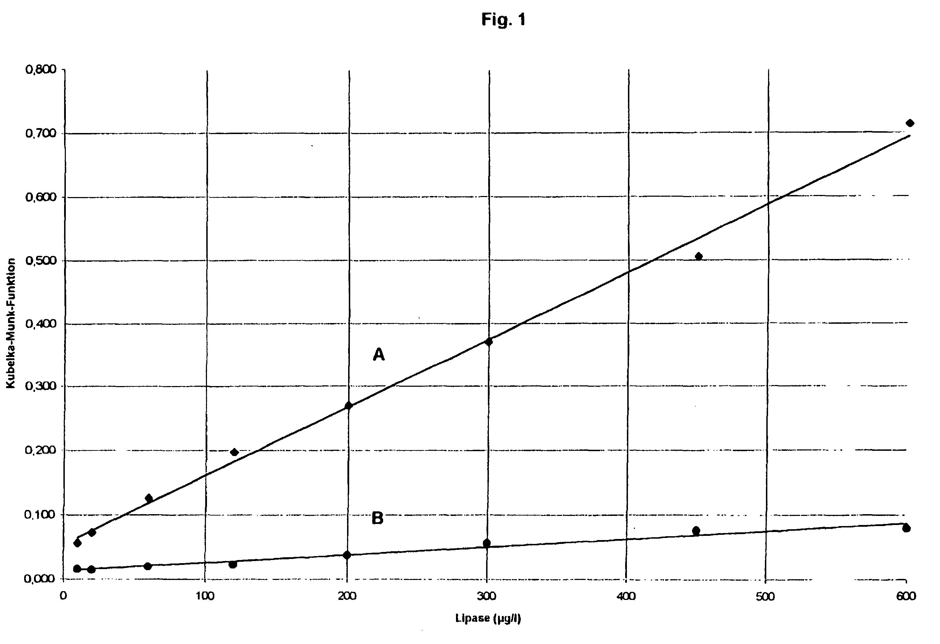

Diese Ergebnisse sind in Fig. 1A graphisch dargestellt. Die gemessenen Remissionswerte wurden nach dem Verfahren von Kubelka und Munk (Z. Tech. Physik 12, 593 (1931)) entsprechend der Gleichung F = (1-R)²/2R (Kubelka-Munk-Funktion) umgerechnet, um eine lineare Beziehung zur Enzymkonzentration zu erhalten. Die erhaltene Gerade entspricht einer linearisierten Standardkurve mit einer Steigung von y = 0,0011x.

### e) Vergleichsversuch

Nach dem Stand der Technik sind sowohl Substrat als auch Oxidationsmittel zusammen auf einem saugfähigen Träger aufgebracht. Ein Filterpapier wird mit einer Lösung von 56 mg 5-Brom-4-chlor-3-indoxylcaprylat und 112 mg Nitroblautetrazoliumchlorid in 112 ml Ethanol getränkt und getrocknet. Das erhaltene Testpapier wird für etwa 2 Sekunden in die Standardlösungen nach c) eingetaucht, abgeschüttelt und 15 Minuten bei Raumtemperatur inkubiert und entsprechend d) ausgewertet:

| | | | | | | | | |
|---|---|---|---|---|---|---|---|---|
| Konz. (µg/l) | 10 | 20 | 60 | 120 | 200 | 300 | 450 | 600 |
| relative Rem. (%) | 86 | 80,9 | 79,2 | 75 | 77,1 | 73,5 | 67 | 56,4 |

Das Ergebnis ist in Fig. 1 B graphisch dargestellt mit dem Resultat, daß die Gerade eine Steigung von y = 0,0001 hat, d.h., daß das Verfahren nach der Erfindung um den Faktor 11 empfindlicher ist als das Verfahren nach dem Stand der Technik. Analoge Ergebnisse werden erhalten, wenn man anstelle von 5-Brom-4-chlor-3-indoxylcaprylat, 5-Brom-3-indoxylcaprylat, 6-Chlor-3-indoxylcaprylat, 5-Brom-6-chlor-3-indoxylcaprylat oder die entsprechenden -palmitate oder -acetate einsetzt.

### Beispiel 2

### Bestimmung von Lipase in Milch

Die Bestimmungen werden analog Beispiel 1 durchgeführt, wobei die Lipasestandardlösung anstelle der Pufferlösung mit pasteurisierter Vollmilch (Fettgehalt 3,5 %) verdünnt wird. Die Ergebnisse der Messung mit dem Reflektometer zeigt die nachstehende Tabelle:

| | | | | | | | | | |
|---|---|---|---|---|---|---|---|---|---|
| Konz. (µg/l) | 10 | 20 | 60 | 120 | 200 | 300 | 450 | 600 | |
| relative | 75,1 | 69,7 | 63,8 | 54,1 | 43,7 | 38,8 | 36,1 | 31,8 | (A) |
| Rem. (%) | 84,0 | 83,5 | 81,5 | 74,1 | 73,5 | 72,5 | 66,5 | 47,3 | (B) |

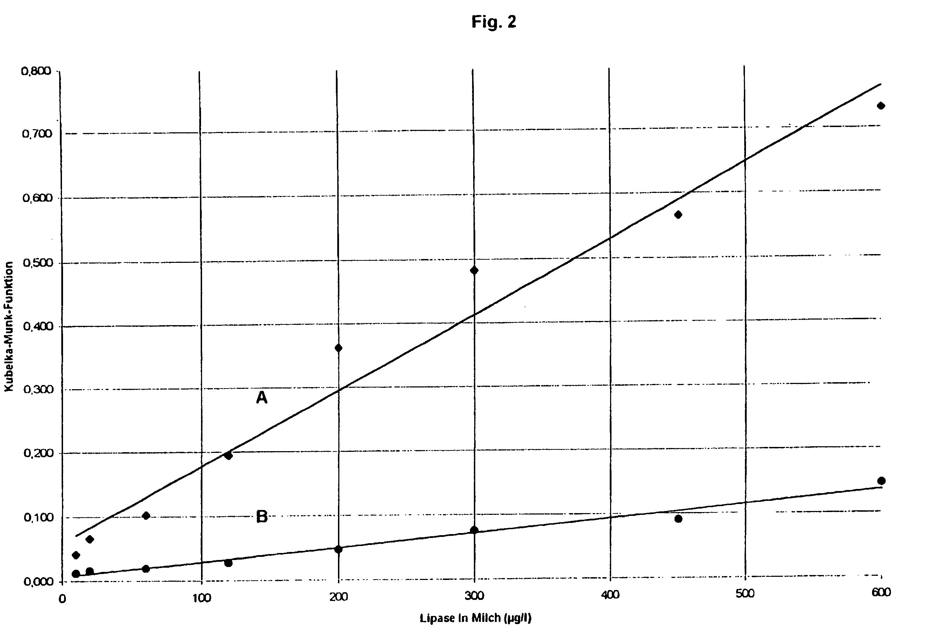

Die Ergebnisse sind in Fig. 2 graphisch dargestellt, wobei die Gerade A die Ergebnisse nach der Erfindung und die Gerade B die Ergebnisse nach dem Stand der Technik wiedergibt. Aus den Steigungen der beiden Geraden (y = 0,0012x und y = 0,0002x) ist zu ersehen, daß das Verfahren nach der Erfindung 6mal empfindlicher ist als nach dem Stand der Technik.

### Beispiel 3

### Bestimmung von Lipase in Serum

Die Bestimmungen werden analog Beispiel 1 durchgeführt, wobei die Lipasestandardlösung mit 0, 10, 20, 50, 100, 200, 500 und 1000 µg/l Lipase anstelle der Pufferlösung mit Humanserum, das 10 Minuten auf 65 °C erhitzt wird, verdünnt wird. Das Ergebnis ist in der nachstehenden Tabelle wiedergegeben. Es zeigt, daß noch 2 µg/l Lipase sicher nachgewiesen werden können.

| Lipase in Serum (µg/l) | **Teststäbchen** | | naßchemischer Test (U/l) |
|---|---|---|---|
| | R (% Remission) | Ro-R (% Remission) | |
| 0 | Ro = 59,9 | **0,0** | **0** |
| 1 | 59,7 | **0,2** | **0** |
| 2 | 57,3 | **2,4** | **0** |
| 5 | 56,3 | **3,4** | **0** |
| 10 | 53,6 | **6,1** | **0** |
| 20 | 48,9 | **10,8** | **0** |
| 50 | 39,2 | **20,5** | **4** |
| 100 | 30,2 | **29,5** | **67** |
| 200 | 21,8 | **37,9** | **156** |
| 500 | 13,4 | **46,3** | **305** |
| 1000 | 9,0 | **50,7** | **753** |

Die Ergebnisse nach der Erfindung (Spalte 3) werden verglichen mit einem im Handel erhältlichen photometrischen, naßchemischen Lipasetest (Spalte 4). Die Aktivität wurde an einem klinisch-chemischen Analyzer gemessen. Das Ergebnis zeigt, daß erst ab 50 µg/l Lipase ein von Null verschiedener Wert gefunden wird. Das erfindungsgemäße Verfahren ist somit etwa 25mal empfindlicher als der handelsübliche Lipasetest.

### Beispiel 4

### Bestimmung von Alkalischer Phosphatase

### a) Herstellung des Testpapiers

Ein Filterpapier (Schöller & Hösch 300A) wird mit einer Lösung von 56 mg 5-Brom-4-chlor-3-indoxylphosphat in 112 ml Ethanol (96 %) getränkt und nach der Tränkung getrocknet. Das erhaltene Papier wird auf ein Trägermaterial, z.B. Polyesterfolie, in bekannter Weise aufgebracht.

### b) Herstellung der Oxidationsmittellösung

Die Oxidationsmittellösung enthält 60 mg Nitroblautetrazoliumchlorid, das in 9,9 g Ethanol (96 %) gelöst wird. Diese Lösung wird zu 49,3 ml Wasser gegeben, das 2 % Detergens (Tween 20) enthält.

### c) Herstellung der Alkalischen Phosphatase-Standardlösungen

Mit Alkalischer Phosphatase aus Kälberdarm werden durch Verdünnen mit einer Pufferlösung enthaltend
1 mol/l Diethanolamin, pH 9,8 und
0,5 mmol/l Magnesiumchlorid

Standardlösungen in den Konzentrationen 0,1, 0,2, 0,5, 1, 2, 5 und 10 µg/l hergestellt.

### d) Bestimmung der Alkalischen Phosphatase

Das Teststäbchen wird für etwa 2 Sekunden in die Enzym eingetaucht, abgeschüttelt und 15 Minuten in etwa 1 ml der Oxidationsmittellösung eingetaucht. Nach dieser Zeit hat sich eine blaue Farbe entwickelt, die in einem Reflektometer (576 nm) gemessen wird:

| | | | | | | | | |
|---|---|---|---|---|---|---|---|---|
| Konz. (µg/l) | 0 | 0,1 | 0,2 | 0,5 | 1 | 2 | 5 | 10 |
| relative Rem. (%) | 74,5 | 73,8 | 73,4 | 71,2 | 67 | 62,4 | 48,5 | 34,4 |

### e) Vergleichsversuch

Nach dem Stand der Technik sind sowohl Substrat als auch Oxidationsmittel zusammen auf einem saugfähigen Träger aufgebracht. Ein Filterpapier wird mit einer Lösung von 56 mg 5-Brom-4-chlor-3-indoxylphosphat und 112 mg Nitroblautetrazoliumchlorid in 112 ml Ethanol getränkt und getrocknet. Das erhaltene Testpapier wird in die Standardlösungen nach c) eingetaucht, abgeschüttelt und 15 Minuten flach liegend bei Raumtemperatur inkubiert. Danach hat sich eine blaue Farbe entwickelt, die im Reflektometer gemessen wird.

| | | | | | | | | |
|---|---|---|---|---|---|---|---|---|
| Konz. (µg/l) | 0 | 0,1 | 0,2 | 0,5 | 1 | 2 | 5 | 10 |
| relative Rem. (%) | 76,8 | 75,7 | 76,1 | 74,5 | 73,1 | 71,2 | 61,5 | 54,5 |

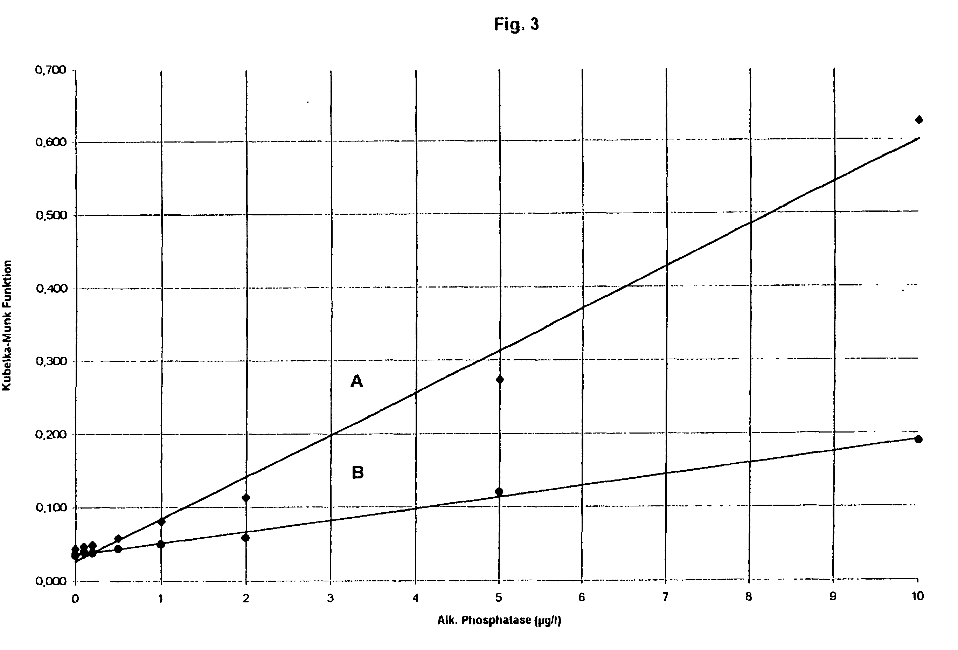

Die Ergebnisse aus d) und e) sind in der Fig. 3 graphisch dargestellt. Dabei wurden die Remissionswerte nach dem Verfahren von Kubelka-Munk entsprechend der Gleichung F = (1-R)²/2Rumgerechnet und damit eine lineare Beziehung zum Enzymgehalt erhalten. Die Gerade A gibt die Ergebnisse nach der Erfindung und die Gerade B die Ergebnisse nach dem Stand der Technik wieder. Aus den Steigungen der beiden Geraden (y = 0,0574x und y = 0,0157x) ist zu ersehen, daß das Verfahren nach der Erfindung 3,6mal empfindlicher ist als das Verfahren nach dem Stand der Technik.

### Beispiel 5

### Bestimmung von Esterasen

Analog Beispiel 4 wird ein Testpapier hergestellt, das mit einer Lösung von 56 mg N-Methyl-indoxylacetat in 112 ml Ethanol (96 %) getränkt wird. Die Oxidationsmittellösung entspricht der in Beispiel 4d). Die Esterase-Standardlösungen in den Konzentrationen 1, 2, 5, 10, 20, 50 und 100 µg/l Esterase aus Schweineleber werden durch Verdünnen mit einer Pufferlösung enthaltend

| | |
|---|---|
| 20 mmol/l | Tris/HCl-Puffer, pH 7,6 |
| 4 mmol/l | Magnesiumchlorid |
| 2 mmol/l | EDTA und |
| 0,1 mg/ml | Rinderserumalbumin |

hergestellt.

Das Teststäbchen wird für etwa 2 Sekunden in die Enzymlösung eingetaucht, abgeschüttelt und 15 Minuten in etwa 1 ml der Reaktionslösung eingetaucht. Nach dieser Zeit hat sich eine blaue Farbe entwickelt, die in einem Reflektometer (576 nm) gemessen wird.

| | | | | | | | | |
|---|---|---|---|---|---|---|---|---|
| Konz. (µg/l) | 0 | 1 | 2 | 5 | 10 | 20 | 50 | 100 |
| relative Rem. (%) | 78,8 | 75,5 | 72,1 | 71,8 | 67,4 | 59,6 | 44,1 | 32,3 |

Zum Vergleich wurde mit einem Testpapier gearbeitet, das mit einer Lösung von 56 mg N-Methyl-indoxylacetat und 112 mg Nitroblautetrazoliumchlorid in 112 ml Ethanol getränkt wird, mit folgendem Ergebnis:

| | | | | | | | | |
|---|---|---|---|---|---|---|---|---|
| Konz. (µg/l) | 0 | 1 | 2 | 5 | 10 | 20 | 5 | 100 |
| relative Rem. (%) | 77,1 | 73,8 | 77 | 75,5 | 74,1 | 71,0 | 65,1 | 57,9 |

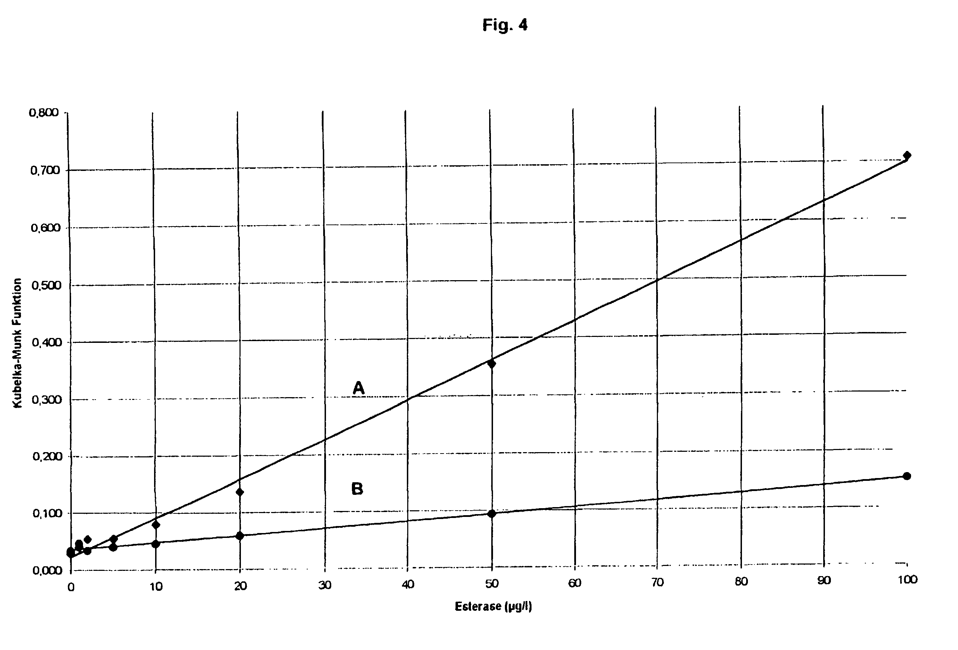

Die Ergebnisse aus beiden Versuchen sind in Fig. 4 graphisch dargestellt. Die Gerade nach dem erfindungsgemäßen Verfahren (A) hat eine Steigung von y = 0,0068x, die des Vergleichsversuchs hat eine Steigung von y = 0,0012x. Das bedeutet, daß das erfindungsgemäße Verfahren um den Faktor 5,7 empfindlicher ist als das Verfahren nach dem Stand der Technik.

## Patentansprüche

1. Verfahren zur Bestimmung von hydrolytischen Enzymen in flüssigen Proben, **gekennzeichnet durch** folgende Verfahrensschritte:
a) Benetzen eines mit einer Indoxylverbindung imprägnierten saugfähigen Trägers mit der Probelösung;
b) Eintauchen des Trägers aus Schritt a) in eine gesonderte, ein Oxidationsmittel enthaltende Lösung;
c) Inkubation des Trägers;
d) reflektometrische Auswertung des Trägers;

2. Verfahren nach Anspruch 1, **dadurch gekennzeichnet, dass** die in Schritt b) verwendete Lösung als Oxidationsmittel eine Tetrazoliumverbindung enthält.

3. Verfahren nach Anspruch 1 oder 2, **dadurch gekennzeichnet, dass** in Schritt a) ein mit Indoxylacetat, -butyrat, -caprylat, -palmitat, -glucuronid, -galactosid, -maltotriose, -phosphat oder - sulfat imprägnierter Träger eingesetzt wird.

4. Mittel zur Bestimmung von hydrolytischen Enzymen in flüssigen Proben nach dem Verfahren entsprechend Anspruch 1, bestehend aus
- einem saugfähigen Träger, der mit einer Indoxylverbindung imprägniert ist, sowie
- einer Lösung, die ein Tetrazoliumsalz als Oxidationsmittel enthält.

5. Mittel nach Anspruch 4, **dadurch gekennzeichnet, dass** die Indoxylverbindung Indoxylacetat, -butyrat, -caprylat, -palmitat, -glucuronid, -galactosid, -maltotriose, -phosphat oder- sulfat ist.

## Claims

1. Procedure for the determination of hydrolytic enzymes in liquid samples, **characterized by** the following steps:
a) wetting an absorbent carrier impregnated with an indoxyl compound with the sample solution;
b) immersing the carrier from step a) in a separate solution comprising an oxidizing agent;
c) incubating the carrier;
d) analysing the carrier by reflectometry.

2. Procedure according to Claim 1, **characterized in that** the oxidizing agent in step b) is a tetrazolium compound.

3. Procedure according to Claim 1 or 2, **characterized in that** the carrier used in step a) is impregnated with indoxyl acetate, butyrate, caprylate, palmitate, glucuronide, galactoside, maltotriose, phosphate or sulphate.

4. Composition for the determination of hydrolytic enzymes in liquid samples according to the procedure of Claim 1, consisting of
- an absorbent carrier which is impregnated with an indoxyl compound, and
- a solution which comprises a tetrazolium salt as oxidizing agent.

5. Composition according to Claim 4, **characterized in that** the indoxyl compound is indoxyl acetate, butyrate, caprylate, palmitate, glucuronide, galactoside, maltotriose, phosphate or sulphate.

## Revendications

1. Procédé pour la recherche des enzymes hydrolytiques dans des échantillons liquides, **caractérisé par** les étapes suivantes
a) imprégnation avec la solution de l'échantillon d'un support absorbant imprégné par un composé indoxyle,
b) immersion du support provenant de l'étape a) dans une solution séparée, contenant un agent oxydant,
c) incubation du support,
d) évaluation par réflectométrie du support.

2. Procédé selon la revendication 1, **caractérisé en ce que** la solution utilisée dans l'étape b) contient, comme agent oxydant, un composé tétrazolium.

3. Procédé selon la revendication 1 ou 2, **caractérisé en ce que** l'on utilise dans l'étape a) un support imprégné par l'acétate, butyrate, caprylate, palmitate, glucorinide, galactoside, maltotriose, phosphate ou sulfate d'indoxyle.

4. Composition pour la recherche des enzymes hydrolytiques dans des échantillons liquides par le procédé selon la revendication 1, constituée
- d'un support absorbant, imprégné d'un composé indoxyle et
- d'une solution contenant un sel de tétrazolium comme agent oxydant.

5. Composition selon la revendication 4, **caractérisée en ce que** le composé indoxyle est l'acétate, butyrate, caprylate, palmitate, glucorinide, galactoside, maltotriose, phosphate ou sulfate d'indoxyle.
